# EUROPEAN PATENT APPLICATION

(11) **EP 1 836 996 A1**
(43) Date of publication of application: **26.09.2007**
(21) Application number: 06111376.7
(22) Date of filing: 20.03.2006
(51) Int. Cl.: A61F 2/08

(54) **Implant for securing a flexible piece to bone**

(71) Applicant: Inion Oy, 33520 Tampere (FI)
(72) Inventor: Vuorisalo, Vesa, 33530, Tampere (FI); Koljonen, Jukka, 37120, Nokia (FI); Väänänen, Petteri, 70200, Kuopio (FI); Nurmi, Janne, 37550, Lempäälä (FI); Nuutinen, Juha-Pekka, 33210 Tampere (FI); Ahvenjärvi, Pia, 33730, Tampere (FI)
(74) Representative: Kaukonen, Juha Veikko

(57) **Abstract**

The invention relates to an implant for securing a flexible piece to bone. The implant (100) comprises a first part (1) and a second part (2), a distal end (3) that is to be inserted first in a hole (5) in the bone, and a proximal end (4) that is at the opposite end of the implant (100) in relation to the distal end (3). The first part (1) comprises securing means (6) for securing the implant to said hole (5). The second part (2) comprises a groove (7) that receives the flexible piece to be secured by means of the implant (100). The second part (2) is arranged to be mobile in relation to the first part (1) in the direction of the longitudinal centre line (C) of the implant. The contact surfaces between the first and the second parts (1, 2) comprise wedging surfaces (8) such that as the second part (2) is shifted towards the proximal end (4) of the implant, said securing means (6) move away from said centre line (C).

## Description

### BACKGROUND OF THE INVENTION

The invention relates to an implant for securing a flexible piece to bone, the implant comprising a first part and a second part, a distal end intended for being inserted first in a hole in the bone and a proximal end at the opposite end of the implant in relation to the distal end.

The invention also relates to the use of the implant for securing a flexible piece to the bone.

The invention further relates to the use of the implant in ACL or PCL operations.

The invention still further relates to the use of the implant in a double-bundle reconstruction.

There are known a large number of different implants intended for use in securing various grafts to bone. Perhaps the best-known operation in this line is replacement of a damaged anterior crucial ligament (ACL) by a graft made from a tendon. Other similar operations include replacement of a posterior crucial ligament (PCL) by a graft made from a tendon and other corresponding operations associated with knee therapy. These operations may also relate to a shoulder, an elbow, an ankle or the like.

In said ACL operation holes are drilled in the femur and the tibia and a graft is secured at both ends to the hole using an implant to be fitted in the hole as the securing means. Said implant may be of a screw type, whereby the implant is screwed between the hole wall and the graft. The implant may also be of a plug-in type, a so-called anchor, which can be anchored in the hole either together with the graft or the graft is subsequently secured to the anchor secured to the bone. A wide variety of different variations of both implant types are known.

Screw-type implants have a drawback that screw threads may cause damage to a graft, because they press and chafe the graft against the bone. Another drawback is that the securing of the graft is not always sufficiently firm, but the graft may slip past the implant off the hole. A further drawback is that the screw generally presses the graft against one wall in the hole, and consequently only one side of the graft may attach to the bone. Yet another drawback is that screw mounting is slow to carry out.

Anchor-type implants have a problem that their installation in the hole is often a difficult task that demands force. In that case an implant made of biodegradable material, in particular, may get broken very easily. Another problem is that the anchor attaches poorly to the bone, and thus it may detach from the hole. Yet another problem is that the anchor does not press the graft against the wall in the hole, and consequently the graft's fixation to the bone is slow.

### BRIEF DESCRIPTION OF THE INVENTION

The object of the present invention is to provide a novel and improved implant, by which problems of the known implants will be avoided.

The method of the invention is characterized in that a first part comprises an outer surface having securing means for securing the implant in said hole, and a second part comprises an outer surface, in which there is provided a groove to receive a flexible piece to be secured with the implant, the second part being arranged movable in relation to the first part in the direction of the longitudinal centre line of the implant, and that the contact surfaces between the first and the second parts comprise wedging surfaces such that as the second part is shifted in the direction of the proximal end of the implant said securing means move away from said centre line.

The basic idea of the invention is that the implant comprises two parts and that means for securing the implant to the bone are in the first part, whereas means primarily in contact with the flexible piece to be secured are in the second part.

The invention has an advantage that the implant can be inserted in the mounting hole easily and with little force, whereby the risk of getting broken during mounting is very small. Among other things this results from the fact that the cross-sectional area of the implant is small when inserted in the hole. Another advantage is that the implant's pullout force is strong, because the securing means bite into the bone the firmer the stronger the force by which the flexible piece is pulled off the hole. Yet another significant advantage to be noted is that the flexible piece to be secured is arranged in the groove which, when correctly designed, presses said piece in a manner that will enhance the attachment of the piece in place.

The basic idea of an embodiment of the invention is that a first part comprises two, spaced apart branches which are interconnected with a connecting piece at the distal end of the implant, and that a second part is arranged between the branches such that the branches, the connecting piece and the second part form a transverse opening in the implant, through which opening said flexible piece is to be fitted and that said groove forms part of the inner surface of the opening. There is an advantage that the flexible piece to be secured will be firmly attached to the anchor when passed through the opening and it will not slide past the implant as may occur, when screw-type implants are used, for instance.

The basic idea of an embodiment of the invention is that a first part comprises two, spaced apart branches which are interconnected with a connecting piece at the proximal end of the implant such that the branches may be bent in relation to the centre line of the implant and that the second part is arranged between the branches such that said groove is arranged away from the proximal end and it forms part of the outer surface of the implant. There is an advantage that the flexible piece to be secured will be particularly easily fitted in the groove on the outer surface of the implant either prior to inserting the anchor and the piece into a joint to be operated or inside the joint.

### BRIEF DESCRIPTION OF THE DRAWINGS

In the following some embodiments of the invention are described in greater detail with reference to the attached drawings, in which
Figure 1a is a schematic perspective view of an implant according to the invention arranged in a drill hole in the bone,
Figure 1b is a perspective view of the implant parts of Figure 1a, with the parts detached from one another,
Figure 2a is a schematic side view of the implant of Figure 1 a when mounted,
Figure 2b is a schematic side view of the implant of Figure 2a when secured in the drill hole,
Figure 2c is a cross-sectional view of the implant of Figure 2a in plane A - A,
Figure 3a is a schematic perspective view of a second implant according to the invention arranged in a drill hole in the bone,
Figure 3b is a perspective view of the implant parts of Figure 3a, with the parts detached from one another,
Figure 4a is a schematic side view of the implant of Figure 3a when mounted,
Figure 4b is a schematic lateral section of the implant of Figure 3a when secured in the drill hole,
Figure 4c is a schematic cross-sectional view of the implant of Figure 4a in plane A - A,
Figure 5a is a schematic lateral section of a third implant according to the invention when mounted, and
Figure 5b is a perspective view of the implant parts of Figure 5a with the parts detached from one another.

For the sake of clarity, some embodiments of the invention are depicted in a simplified manner in the figures. Like reference numerals refer to like parts in the figures.

### DETAILED DESCRIPTION OF SOME EMBODIMENTS OF THE INVENTION

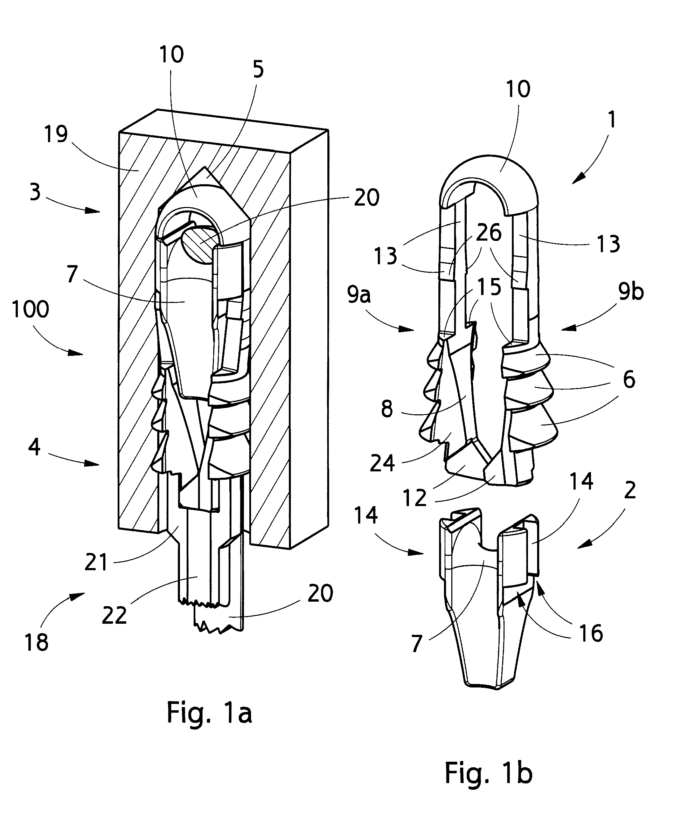

Figure 1a is a schematic perspective view of an implant according to the invention fitted in a drill hole in the bone, and Figure 1 b is a perspective view of the implant parts of Figure 1a, with the parts detached from one another.

The implant 100 is inserted in a hole 5 in the bone 19, the hole being in this case a bottom hole made by drilling. Making a hole 5 in the bone is known per se, and hence it is not discussed in this specification in any greater detail. It should be noted, however, that the hole 5 may also be a through-hole. In ACL or PCL operations the bone 19 is either the femur or the tibia.

The figures only show a part of the bone 19 that is cut into a cylindrical shape with a rectangular bottom.

The implant 100 comprises a first part 1 and a second part 2, which are advantageously made of biodegradable material, whereby the system will break down the implant 100 with time.

The parts 1 and 2 may be manufactured of polymer, copolymer, polymer compound or polymer composite that degrades in the system. The manufacturing material is, for example, a polymer, copolymer, polymer compound or polymer composite of lactic acid, L-lactide, D-lactide, D,L-lactide, mesolactide, glycolic acid, glycolide or the like, and optionally in addition that of another cyclic ester copolymerizable with a lactide. To provide the material with desirable properties the manufacturing material may additionally comprise other comonomers, such as α-, β-, and γ-hydroxy butyric acid, α-, β-, and γ-hydroxy valerianic acid and other hydroxyl fatty acids (C₁₁ - C₂₅), such as stearic acid, palmitinic acid, oleic acid, lauric acid and the like. The manufacturing material may thus be a polylactide, polyglycolide, poly(L-lactide), poly(D-lactide), poly(L-lactide-co-D,L-lactide), poly(L-lactide-co-mesolactide), poly(L-lactide-co-glycolide), poiy(L-iactide-co-e-caproiactone), poly(D,L-lactide-comesolactide), poly(D,L-lactide-co-glycolide), poly(D,L-lactide-co-ε-caprolactone), poly(mesolactide-co-glycolide), poly(mesolactide-co-ε-caprolactone) or the like. For example, poly(L-lactide-co-D,L-lactide) 70:30, poly(L-lactide-co-D,L-lactide) 80:20, poly (L-lactide-co-glycolide) 85:15 and poly(L-lactide-co-glycolide) 80:20 can be given here as suitable copolymeric manufacturing materials.

Moreover, the manufacturing material may also contain trimethylenecarbonate or dioxanone. These manufacturing materials include poly(L-lactide-co-trimethylenecarbonate), poly(D,L-lactide-co-trimethylenecarbonate), poly(mesolactide-co-trimethylenecarbonate), poly(glycol-co-trimethylenecarbonate), poly(L-lactide-co-dioxanone), poly(D,L-lactide-co-dioxanone), poly(mesolactide-co-dioxanone), poly(glycol-co-dioxanone) and the like.

Particularly advantageous manufacturing materials include poly(L-lactide), poly(L-lactide-co-D,L-lactide), poly(L-lactide-co-trimethylenecarbonate) and the blends thereof.

The manufacturing material may contain bioglass, bioceramics, biologically active material or drug, such as antibiotic or growth factor. The biologically active agent may be bone morphogenic proteins (BMP), such as OP-1, BMP-2, BMP-4 and BMP-7. The biologically active agent may be provided in the implant 100, for instance, by adding it to the material from which the parts 1 and 2 are made or by coating one or both of the parts 1, 2 with a coating containing the biologically active agent.

In the manufacturing material it is possible to mix a colorant, such as D&C Green No. 6 having a chemical name 1,4bis[(4-methylphenyl)amino]-9,10-anthrasenedione (CAS No. 128-80-3) or a colorant D&C Violet No. 2, the chemical name of the colorant being 1,4-hydroxy[(4-methylphenyl)amino]-9,10-anthrasenedione (CAS No. 81-48-1). The amount of colorant is advantageously at most 0.03, most preferably 0.002...0.02 % by weight.

The manufacturing material may further contain softening agents, such as pyrrolidone. Useful pyrrolidones include all pyrrolidones known per se, which soften the manufacturing material and which do not cause adverse tissue reactions or toxic reactions. These pyrrolidones include, for instance, N-methyl-2-pyrrolidone (NMP), 1-ethyl-2-pyrrolidone (NEP), 2-pyrrolidone (PB) and 1-cyclohexyl-2-pyrrolidone (CP), NMP and NEP being particularly advantageous examples.

Naturally it is also possible to manufacture the implant 100 of one or more biostable materials, such as plastic, for instance, PEEK (polyetherether ketone) or metal, for instance, titanium.

The first part 1 constitutes the distal end 3 of the implant 100, i.e. the end that is first inserted in the hole 5 as the implant 100 is installed. The first part 1 also constitutes the proximal end 4, which is at the opposite end of the implant 100 in relation to the distal end 3 and to which an instrument used in installing the implant 100 will be fitted.

The first part 1 comprises two branches 9a, 9b that are spaced apart. The branches 9a, 9b are interconnected with a connecting piece 10 at the distal end 3 of the implant.

The first part 1, or either branch 9a, 9b thereof, comprises securing means 6 on its outer surface. In the embodiment of the implant 100 shown Figure 1a the securing means 6 are ridges which have a triangular cross section and which are arranged substantially perpendicularly to the longitudinal centre line C of the implant 100. The securing means 6 may also be designed such that they bite into the bone 19 when the implant is rotated about its longitudinal centre line C. The end of the securing means 6 can be provided to have a shape that cuts or packs the bone, which facilitates the penetration of the securing means 6 into an intact wall of the hole 5 when the implant 100 is rotated. It is possible to rotate the implant 100 with a suitable instrument. The magnitude of the rotational movement is typically about 90 degrees at most. The securing means 6 may also be designed to have the shape of a screw thread such that the second part 2 - and optionally the first part 1 - not only turns about the centre line C by the effect of the rotational movement, but also shifts in the hole 5 in the direction of said centre line C.

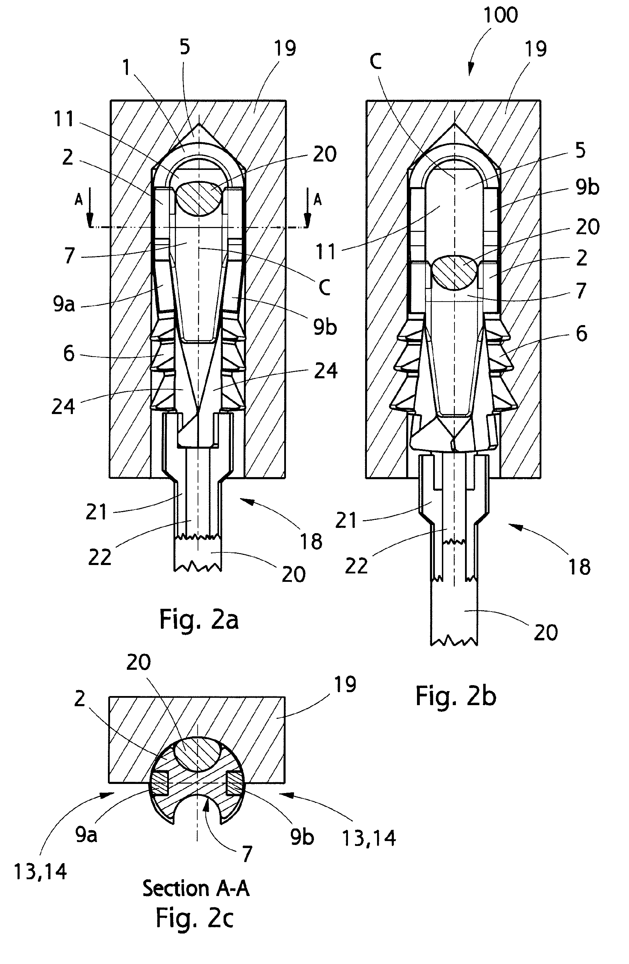

The function and the operation of the securing means 6 are discussed in greater detail in connection with Figures 2a and 2b.

At the end of the second part 2 of the implant 100 that is closer to the distal end 3 of the implant 100 there is provided a groove 7 that forms a saddle-surface-like surface at said end of the second part 2. The groove 7 runs from one side of the second part 2 via said end to the other side of the second part 2. The groove 7 is actually substantially equal in length with the side of the second part.

In this case the flexible piece to be secured with the implant 100 is a tendon 20. The tendon 20 may originate, for instance, from the patient's own body, from somebody else's body, from an animal, it may be an artificial synthetic tendon or a tendon prepared by processes of tissue technology. Moreover, the implant 100 may be used for attaching suture threads to bone 19. It is possible to secure a tendon, soft tissue and so on, to said suture threads.

The tendon 20 is arranged in the implant 100 such that it runs in the groove 7 around the end of the second part 2 through the transverse opening 11 in the implant. Said transverse opening 11 is formed between the branches 9a, 9b of the first part 1, the connecting piece 10 and the second part 2.

In Figure 1a the tendon is cut at the highest point of the groove 7, in order for the structure of the implant to be seen better. Actually the tendon 20 turns back towards the proximal end 4 of the implant 100 following the groove 7 and further out through the hole 5. The ends of the tendon 20, which are not shown in the figure, are attached in a manner known per se either to one another or apart from one another. The attachment of the ends may be implemented by sewing with suture threads or by entwining or by any method known per se.

The second part 2 may move in relation to the first part 1, because sliding surfaces 13 in the branches 9a, 9b of the first part 1 are arranged in longitudinal sliding grooves 14 on the sides of the second part 2. In the embodiment of the implant 100 shown in Figure 1a each branch 9a, 9b comprises three sliding surfaces 13, the outer ones of which are arranged mutually on the opposite sides of the branch 9a, 9b in question and the middle-most sliding surface is perpendicular to the outermost sliding surfaces.

Correspondingly the sliding groove 14 is a groove matching with said three sliding surfaces 13. It is possible to slide the second part 2 in relation to the first part 1 in the longitudinal direction of the implant 100 within the range permitted by the length of the sliding surfaces 13.

The cross section formed by the sliding surfaces 13 and the cross section of the corresponding groove 14 may have some other shape as well. The cross section of the sliding groove 14 may have any suitable concave shape, such as an arcuate surface, or it may resemble a dovetail groove. Correspondingly, the cross section formed by the sliding surfaces 13 may be any convex cross section that fits in the sliding groove. It should further be noted that the sliding surfaces 13 may also be arranged in the second part 2 and the sliding groove 14 in the first part 1 respectively. The shape of the sliding groove 14 and/or the sliding surface 13 need not necessarily be the same for their/its entire length.

It is also possible to arrange the sliding surfaces 13 in just one branch 9a, 9b, whereby the second part 2 comprises a sliding groove 14 on one side only. It is further possible that the branches 9a, 9b have mutually different sliding surfaces.

On the outermost sliding surfaces 13 there are arranged locking means 26, four in all. In the present embodiment the locking means 26 are wedge-like projections, past which the second part 2 can be drawn using very little force from the distal position shown in Figure 2a to the locking position shown in Figure 2b. Instead, the locking means 26 prevent the second part 2 from sliding back to its distal position.

The locking means 26 may also be implemented, for instance, such that in the sliding surface 13 there is arranged a notch or a recess, and in a sliding groove 14 portion sliding on said sliding surface there is arranged a projection that clicks into said recess when the second part 2 is in the locking position.

It should be noted that the locking means 26 may also be only in one of the two branches 9a, 9b and that the implant 100 may also be implemented completely without the locking means 26.

At the ends of the branches 9a, 9b there are designed projections 24 such that when the implant is in the hole 5, before the implant securing means 6 are attached to the wall of the hole 5, said projections 24 form two wedging surfaces 8 on the inner surfaces of the branches 9a, 9b. The wedging surfaces 8 approach the longitudinal centre line C of the implant when seen from the distal end 3 towards the proximal end 4.

The sides of the second part 2 approach one another beneath the sliding grooves 14 in the direction of the proximal end 4 of the implant such that they will come into contact with the wedging surfaces 8 substantially for all their area when the implant 100 is locked in the hole 5. This situation is shown in Figure 2b. However, it is also possible to design the sides of the part 2 in some other manner.

In Figures 1a and 2a the implant is shown in the state of installation, in other words, the implant 100 has been inserted so deep in the hole 5 that the securing of the implant 100 to the bone 19 may be started. The implant 100 has been inserted in the hole 5 with an instrument 18 provided for this purpose, of which instrument only the head closest to the proximal end of the implant 100 is seen in the figure. In this case the instrument 18 includes a contact part 21, which comprises means for engaging to counterparts 12 arranged at the proximal end 4 of the branches 9a, 9b of the second part 2. In the present embodiment the counterparts 12 are juxtaposed in the contact part 21, but naturally this is not necessary.

In this case the instrument further includes an installation head that is movable in relation to the contact part 21.

The implant 100 is secured to the hole 5 by forcing the securing means 6 into the bone tissue 19 in the wall of the drill hole 5. This can be carried out for instance such that the contact part 21 of the instrument 18 is first drawn outwardly from the hole 5 such that the counterparts 12 in the implant are released from its grip. Thereafter, the ends of the tendon 20 are pulled with a sufficient force to make the second part 2 of the implant move towards the proximal end 4 of the implant. Simultaneously the first part 1 of the implant is prevented from being displaced in the pulling direction by supporting it with the instrument 18. The second part 2 of the implant approaching the proximal end 4 wedges between the projections 24 and forces the branches 9a, 9b gradually outwards, i.e. away from the centre line C. At the same time the securing means 6 penetrate into the bone 19.

Tightening of the tendon 20 is continued until counter surfaces 16 of the shoulder of the second part 2 come into contact with the shoulders 15 of the first part, which is the situation shown in Figure 2b. The operator tightening the tendon 20 will detect this as a sudden and considerable rise in tractive resistance. The counter surfaces 16 of the shoulders and the shoulders 15 prevent the second part 2 from sliding past the first part 1 out of the hole 5.

In Figure 2b the implant 100 is shown locked in the hole 5. The implant 100 engages to the bone 19, particularly if the tendon 20 subjects it to traction. The locking means 26 make sure that the second part 2 will not retract in the direction of the distal end 3.

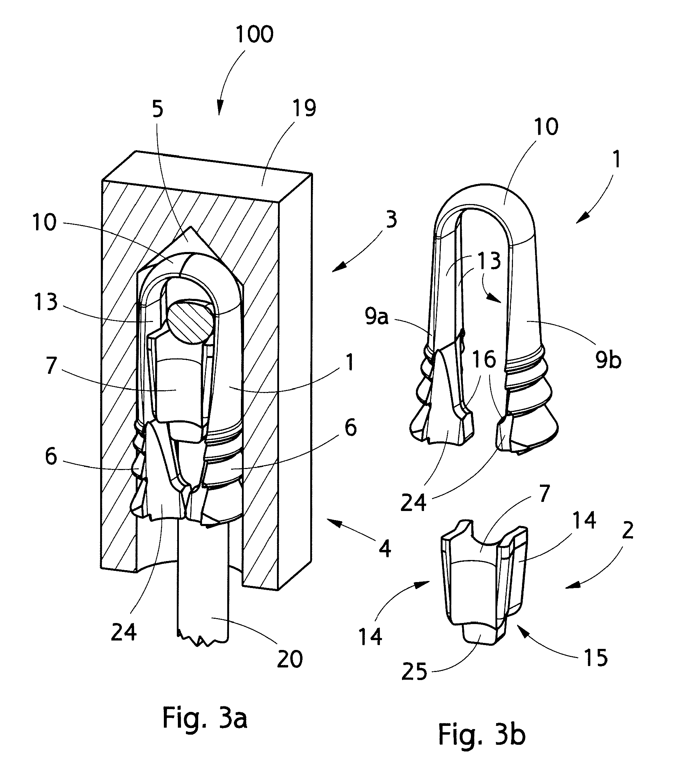

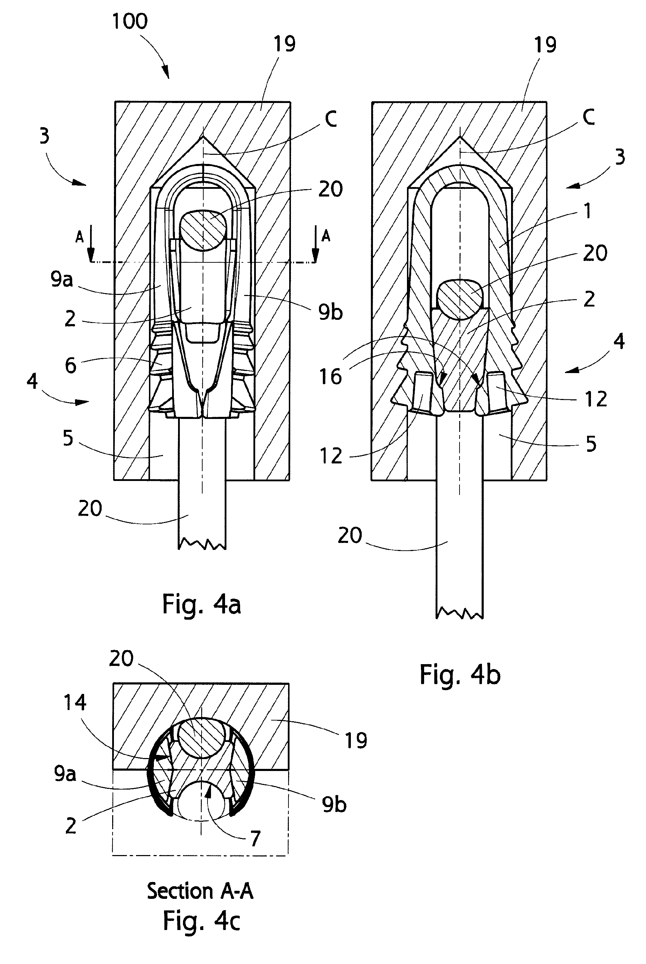

Figure 3a is a schematic perspective view of a second implant according to the invention, the implant being fitted in a drill hole in the bone; Figure 3b shows parts of the same implant detached from one another as a perspective drawing; Figure 4a is a side view of the same implant when mounted; Figure 4b shows a schematic lateral section of the same implant attached to the drill hole; and Figure 4c is a schematic view of the same implant in cross section in plane A - A.

The basic structure of the implant 100 is the same as that of the implant shown in previous figures. There are, however, some differences between said implants. For instance, each branch 9a, 9b now comprises two sliding surfaces 13 instead of three sliding surfaces. In addition the sliding surfaces 13 have been arranged on the inner surfaces of the branches 9a, 9b at an obtuse angle to one another, which shows particularly clearly in Figure 4c. A longitudinal sliding groove 14 provided in the second part 2 is correspondingly a groove having a bottom of a wide V-shape. The sliding surfaces 13 may also be arranged at an acute angle to one another.

Further, the counter surfaces 16 of the shoulder and the shoulders 15 are placed such that the counter surface 16 is now the upper surface of the projections 24 in the first part 1, and correspondingly, the shoulder 15 is at the sliding groove's 14 end that is closest to the proximal end 4 of the implant.

For the installation instrument, which is not shown in Figures 3a - 4c, at the proximal end of the branches 9a, 9b there are provided counterparts 12, which in this embodiment are recesses having a round, oval or polygonal cross-section. When the implant 100 is attached to the instrument the recesses are substantially parallel with both one another and the longitudinal centre line C. When necessary the implant 100 may be turned in the hole 5 about the longitudinal centre line C with an instrument 18 fitted in the counterparts 12.

At the installation stage the projection 25 contributes to wedge the branches 9a, 9b outwardly and presses the securing means 6 of the attached implant 100 into the bone 19.

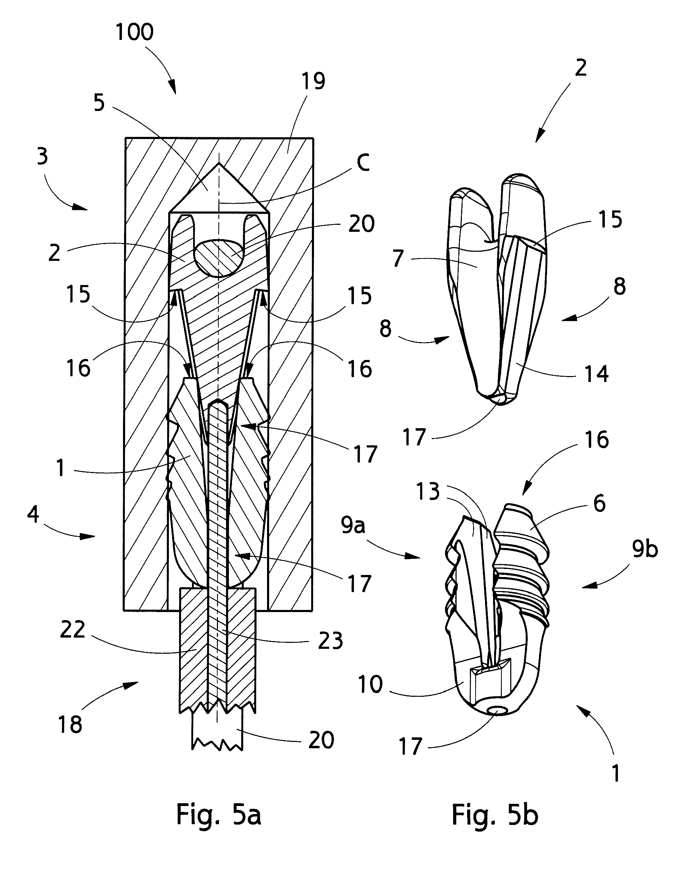

Figure 5a shows a schematic lateral section of a third implant according to the invention in an installation situation and Figure 5b is a perspective view of the parts of the implant in Figure 5a with the parts detached from one another.

The implant 100 comprises a first part 1 and a second part 2 like the implants in the previous figures. However, the first part 1 is now arranged vice versa: its branches 9a, 9b open towards the distal end 3 and the connecting piece 10 between them is at the proximal end 4 of the implant 100. The first part 1 comprises on its outer surface securing means 6, by means of which the implant 100 is secured to its securing hole 5. In this connection it should be noted that in most cases the securing hole 5 is a hole drilled particularly for the securing of the implant 100, but it may also be any suitable hole in the bone 19. The second part 2 comprises a groove 7 for receiving a flexible piece, the groove 7 being located in the installation-ready implant 100, as shown in Figure 5a, at the distal end 3 thereof. The groove 7 extends to both sides of the second part 2 and is equal in length with the entire second part 2. However, the groove 7 need not necessarily be equal in length with the entire second part 2, but in another embodiment of the implant it may be shorter, to the extent that in practice it will not extend to the sides of the second part 2 at all.

The second part 2 tapers in the direction of the proximal end 4 of the implant 100 and on the sides thereof there are wedging surfaces 8. The wedging surfaces 8 are provided with longitudinal sliding grooves 14, both of which are formed of two sliding surfaces arranged at an angle to one another. Correspondingly, on the inner surfaces of the branches 9a, 9b of the first part 1 there are sliding surfaces 13 that fit in the sliding grooves 14 of the second part 2.

The end of the second part 3, which is locating on the side of the proximal end 4 of the implant 100, is arranged between the branches 9a, 9b of the first part 1 in the installation-ready implant 100 such that the groove 7 forms part of the outer surface of the distal end 3 of the implant 100. In Figure 5a a tendon 20 that curves around the distal end 3 of the implant in said groove 7 is arranged in the groove 7. The deep groove 7 protects the tendon 20 and gives it space to run between the wall of the hole 5 and the implant 100. The groove 7 that is correctly designed for the tendon 20 presses the tendon 20 against the bone 19 such that the blood circulation in the tendon 20 may be restored and function optimally, and yet the contact of the tendon 20 to the bone 19 is good in view of its fixation. The implant 100 or the second part 2 thereof may be manufactured in a plurality of variations that mainly differ just in the measurements of the groove 7. This is how optimally measured implants 100 are obtained for tendons 20 of different sizes.

A further advantage of a long, saddle-surface-like groove 7 is that the implant 100 has as few edges as possible, or no edges at all, that could damage the tendon 20 during or after the installation of the implant 100.

On the longitudinal centre line C of the implant 100 there is arranged a guiding hole 17 that consists of a part extending through the first part 1 and a bottom hole locating in the second part 2. In the guiding hole 17 there is arranged a guiding pin 23 belonging to the instrument 18. The guiding pin 23 arranged in the guiding hole 17 ensures that the implant parts 1, 2 stay in a correct mutual position on inserting the implant 100 in the hole 5 and/or on securing the implant 100 to the bone 19. The guiding hole 17 shown in the figures is round in cross section, but alternatively it may be non-round, for instance, oval or polygonal. A non-round guiding hole 17 allows the operator to turn the implant 100 that is fitted but not yet secured in the hole 5 about the longitudinal centre line C. Naturally the guiding hole 17 may also be arranged in the embodiments of the implant 100 represented in the previous figures. It should be noted, however, that the guiding hole 17 is not a necessary feature in the implant 100.

The implant 100 is secured to the bone 19 such that the second part 2 is forced to penetrate between the branches 9a, 9b by pulling the tendon 20 and simultaneously by resisting with the instrument 18 the motion of the first part 1 off the hole 5. The guiding pin 23 is able to slide out of the hole 5 in relation to the installation head 22 of the instrument 18 as the second part 2 advances, or the guiding pin 23 may be withdrawn in advance from at least the portion of the guiding hole 17 in the second part 2. The second part 2 presses the ridges serving as the securing means 6 of the first part 1 against the bone 19, into which the implant 100 is thus attached.

At the end of both branches 9a, 9b there is arranged a counter surface 16 for the shoulder, which serves as the counter surface for the shoulders 15 on the sides of the second part 2. The motion of the second part 2 in relation to the first part 1 stops at the latest when the shoulders 15 come into contact with the counter surfaces 16 of the shoulders.

In some cases features set forth in this document may be used as such, irrespective of other features. On the other hand, features set forth in this document may be combined, when necessary, so as to provide various combinations.

The drawings and the specification relating thereto are only intended to illustrate the inventive idea. The details of the invention may vary within the scope of the claims. The first part 1 may be made of material different from the second part 2. In one embodiment of the invention the first part is made of biodegradable material whereas the second part is made of biostable material. The implant 100 may also be used in a so-called double-bundle reconstruction, which is sometimes called an anatomical reconstruction. In that case the second part 2 of the implant, or in case the implant 100 as shown in Figures 5a, 5b is concerned, the first part 1 is made of biostable material. The biostable first or second part keeps the tendon 20 fibres locating on different sides thereof apart from one another, whereby said double-bundle reconstruction is achieved. Because the biostable part does not disintegrate in the body said reconstruction is permanent. The double-bundle reconstruction is used, for instance, in ACL reconstructions or other similar reconstructions known per se.

## Claims

1. An implant for securing a flexible piece to bone, the implant (100) comprising:
a first part (1) and a second part (2),
a distal end (3) intended for being inserted first in a hole (5) in the bone and
a proximal end (4) at the opposite end of the implant (100) in relation to the distal end (3), **characterized in that**
the first part (1) comprises an outer surface including securing means (6) for securing the implant in said hole (5), and
the second part (2) comprises an outer surface, in which there is provided a groove (7) to receive a flexible piece to be secured by means of the implant (100),
the second part (2) being arranged movable in relation to the first part (1) in the direction of the longitudinal centre line (C) of the implant, and that
the contact surfaces between the first and the second parts (1, 2) comprise wedging surfaces (8) such that
on shifting the second part (2) in the direction of the proximal end (4) of the implant said securing means (6) move away from said centre line (C).

2. The implant of claim 1, **characterized in that**
the first part (1) comprises two, spaced apart branches (9a, 9b) which are interconnected with a connecting piece (10) at the distal end (3) of the implant, and that
the second part (2) is arranged between the branches (9a, 9b) such that the branches (9a, 9b), the connecting piece (10) and the second part (2) form a transverse opening (11) of the implant, through which opening said flexible piece is to be fitted and that
said groove (7) forms part of the inner surface of the opening (11).

3. The implant of claim **1, characterized in that**
the first part (1) comprises two, spaced apart branches (9a, 9b) which are interconnected with a connecting piece (10) at the proximal end (4) of the implant such that the branches (9a, 9b) may be bent in relation to the centre line (C) of the implant and that
the second part (2) is arranged between the branches (9a, 9b) such that said groove (7) is arranged away from the proximal end (4) and it forms part of the outer surface of the implant (100).

4. The implant as claimed in any one of the preceding claims, **characterized in that** said securing means (6) are projections.

5. The implant of claim 4, **characterized in that** the projections are ridges arranged at an angle to the longitudinal centre line (C).

6. The implant of claim 4 or 5, **characterized in that** the projections have an asymmetrical cross section such that the angle between the surface of the projection and the longitudinal centre line (C) is more abrupt on the proximal end (4) side.

7. The implant as claimed in any one of the preceding claims, **characterized in that** the connecting piece (10) is flexible to allow adjustment of the distance between the branches (9a, 9b).

8. The implant as claimed in any one of the preceding claims, **characterized in that** the inner surface of the branches (9a, 9b) forms a space between the branches (9a, 9b) that tapers in the direction of the proximal end (4).

9. The implant as claimed in any one of the preceding claims, **characterized in that** the groove (7) continues on the sides of the second part (2).

10. The implant as claimed in any one of the preceding claims, **characterized in that** the groove (7) forms a saddle surface.

11. The implant as claimed in any one of claims 2 to 10, **characterized in that** at least one branch (9a, 9b) comprises a longitudinal sliding surface (13), and correspondingly, the second part (2) comprises a longitudinal surface on which the sliding surface (13) slides as the second part (2) is shifted in the direction of the proximal end (4) of the implant.

12. The implant of claim 11, **characterized in that** said branch (9a, 9b) comprises at least two sliding surfaces (13) that are arranged at an angle to one another.

13. The implant of claim 11 or 12, **characterized in that** the sliding surface (13) is arranged in the branch (9a, 9b).

14. The implant of claim 11 or 12, **characterized in that** the sliding surface (13) is arranged in the second part (2).

15. The implant as claimed in any one of claims 11 to 14, **characterized in that** the sliding surface (13) comprises a locking means (26), which is arranged to lock the second part (2) in relation to the first part (1).

16. The implant as claimed in any one of the preceding claims, **characterized in that** in one part (1, 2) there is a shoulder (15) and in another part there is a shoulder counter surface (16) arranged such that as the shoulder (15) comes into contact with the shoulder counter surface (16) the motion between the first and the second parts (1, 2) stops in the hole (5).

17. The implant as claimed in any one of the preceding claims, **characterized by** being made at least in part of biodegradable material.

18. The implant as claimed in any one of claims 1 to 16, **char-acterized by** being made at least in part of biostable material.

19. The implant as claimed in any one of the preceding claims, **characterized in that** the implant (100) comprises a guiding hole (17) which is parallel with the longitudinal centre line (C) and which extends to both the first and the second parts (1, 2).

20. The implant of claim 19, **characterized in that** the cross section of the guiding hole (17) is round.

21. The implant of claim 19, **characterized in that** the cross section of the guiding hole (17) is non-round such that the implant (100) may be rotated about the longitudinal centre line (C) with an instrument fitted in the guiding hole (17).

22. The implant as claimed in any one of the preceding claims, **characterized in that** it comprises counterparts (12) for the instrument (18) and that the implant (100) may be rotated in the hole (5) about its longitudinal centre line (C) with the instrument (18) fitted in the counterparts (12).

23. Use of the implant as claimed in any one of claims 1 to 23 for securing a flexible piece to the bone (19).

24. Use of the implant as claimed in any one of claims 1 to 23 for ACL or PCL operations.

25. Use of the implant as claimed in any one of claims 1 to 24 for a double-bundle reconstruction.
